# EUROPÄISCHE PATENTSCHRIFT

(11) **EP 0 833 682 B1**
(45) Veröffentlichungstag und Bekanntmachung des Hinweises auf die Patenterteilung: **19.10.2005**
(21) Anmeldenummer: 97919375.2
(22) Anmeldetag: 17.04.1997
(51) Int. Cl.: A61N 2/02

(54) **SPULENMATTE**
COIL MAT
MAT DE BOBINES

(30) Priorität: 19.04.1996 DE 19615647
(43) Veröffentlichungstag der Anmeldung: 08.04.1998
(73) Patentinhaber: Hartmann, Heide, 80804 München (DE)
(72) Erfinder: KÖNIG, Herbert, D-80804 München (DE)
(74) Vertreter: Schlenk, Manfred
(86) Internationale Anmeldenummer: PCT/EP1997/001914
(87) Internationale Veröffentlichungsnummer: WO 1997/039800

(56) Entgegenhaltungen:
- EP-A- 0 239 098
- WO-A-93/00960
- DE-A- 3 344 471
- US-A- 4 825 162

## Beschreibung

Die Erfindung betrifft ein Gerät zur Erzeugung eines flächenhaften Magnetfeldes mit einer an einen Generator zur Stromspeisung angeschlossenen Spulenmatte aus nebeneinander über die Mattenfläche verteilten, in Serien- und/oder Parallelschaltung verbundenen Einzelspulen. Solche Spulenmatten werden vor allem für die magnetische Wechselfeld-Beeinflussung von biologischem Material und Gewebe eingesetzt, beispielsweise um den Transport von Ionen von dem eine Zelle umgebenden Gewebe in das Zellinnere zu verstärken.

Geräte für solche Zwecke nebst den zugehörigen Spulenmatten sind beispielsweise durch die Internationale Patent-Anmeldung W 93/00960 und den in deren Recherchenbericht zitierten Stand der Technik bekannt. Mittels magnetischer Felder, insbesondere solchen mit besonderem zeitlichem Verlauf ihrer Amplitude, lassen sich nämlich biologische Wirkungen erzielen. Zur Erzeugung dieser Felder sind von einem entsprechenden Generator, wie einem Funktionsgenerator gespeiste Spulen vorgesehen. Die Ausbildung der Spulen erfolgt meist als Zylinderspule, deren Innenbereich als Wirkungsbereich dient oder als spiralförmige Flachspule, deren Aussenraum als Wirkungsbereich dient. Auch ist es bekannt, mehrere solche Flachspulen über eine vorgegebene Fläche verteilt vorzusehen und in eine sogenannte Behandlungsdecke einzubringen. Diesen Spulen ist hinsichtlich des mit ihnen erzeugbaren Magnetfeldes gemeinsam, daß dieses im räumlichen Wirkungsbereich störend große Feldstärkeunterschiede aufweist. Eine für solche Spulen bekannte Aufteilung der Fläche in eine größere Anzahl von Teilflächen, deren jede eine Leiterspirale beinhaltet, verändert diese magnetischen Verhältnisse nicht grundlegend (vergleiche z.B. die Deutschen Offenlegungschriften 4004682 A1 und 4108437 A1). Ähnliche Schwierigkeiten treten bei Zylinderspulen auf, wenn man das magnetische Feld im Innenraum betrachtet. Hinzukommt, daß die elektrischen Zuleitungen zu den Anschlüssen der einzelnen Spule weitere, unerwünschte Spitzenwerte des magnetischen Feldes zur Folge haben können. Um ein gleichförmigeres Magnetfeld zu erreichen, wurde auch die Verwendung eines Helmholtz-Spulenpaares empfohlen, dessen innerer Feldraum als Wirkungsbereich benutzt wird. Diese Lösung ist wegen der bekannten Bedingung von wenigstens angenäherter Übereinstimmung von Spulendurchmesser und Spulenabstand indes räumlich ebenso sperrig und aufwendig wie Zylinderspulen und deshalb nur in wenigen Fällen anwendbar.

Diesen Schwierigkeiten wird, ausgehend von einem Gerät zur Erzeugung eines flächenhaften Magnetfeldes mit einer an einen Generator angeschlossenen Spulenmatte aus nebeneinander über die Mattenfläche verteilten, in Serien- und/oder Parallelschaltung verbundenen Einzelspulen dadurch begegnet, daß die mit dem Generator verbundenen Einzelspulen als untereinander wenigstens nahezu flächeninhaltsgleiche Leiterschleifen ausgebildet sind, die zumindest angenähert Rechteckform haben, und dass die zumindest angenähert Rechteckform habenden Leiterschleifen sich teilweise überlappend und mit ihren entsprechenden Rechteckseiten zueinander parallel, über die Mattenfläche gleichförmig verteilt angeordnet und mit einer, den Generator enthaltenden Schaltung zur Speisung verbunden sind

Weitere Einzelheiten und Weiterbildungen der Erfindung sind der Beschreibung und den Patentansprüchen zu entnehmen.

Durch die US-Patentschrift 4825162 ist es für den Empfang sogenannter NMR-Antwortsignale bei Tomographen bekannt, mehrere getrennte Empfänger vorzusehen und jedem der Empfänger eine eigene Leiterschleife zuzuordnen. Durch eine überlappenden Anordnung der einzelnen Leiterschleifen soll sich nach den dortigen Ausführungen über ein gewisses räumliches Erfassungsgebiet ein gleichförmigeres SNR (Signal zu Geräusch-Verhältnis) erreichen lassen. Abgesehen davon, daß dies eine andere Aufgabenstellung als bei der Erfindung ist, ist diese Methode auch nicht in der dort aufgezeigten Weise auf den Fall der einer Behebung von örtlichen Feldkonzentrationen, insbesondere von Feldstärkespitzen übertragbar.

In der Deutschen Offenlegungsschrift 3344471 A1 wird zur Erzielung eines gleichförmigen Magnetfeldes über eine Fläche eine Lösung dahingehend empfohlen, einen Abschnitt einer Leiterschleife als ein metallisiertes textiles Flächengebilde auszubilden und die Technik massiver Leiter zu verlassen. Allerdings zeigen diese Anordnungen trotzdem hohe Feldkonzentrationen im Bereich des nichttextilen Rückleiters, sodaß das textile Flächengebilde wesentlich größer in seinen Abmessungen sein muß, als die Fläche, in der eine Beeinflussung durch das zu erzeugende Magnetfeld erfolgen soll. Über die Haltbarkeit solcher metallisierter Flächengebilde bei Biegebeanspruchungen sind keine Aussagen entnehmbar. Diese ist aber wesentlich, wenn über lange Zeit konstante Feldverhältnisse reproduzierbar sein sollen, weil Metallisierungsunterbrechungen zu abweichenden Stromverteilungen führen. Ferner ist dort angegeben, daß sich mit mehreren solchen textilen Flächengebilden, je nach der gegenseitigen Anordnung, Ausrichtung und Speisung der textilen Flächengebilde, magnetische Wanderfelder oder Rotationsfelder erzeugen lassen.

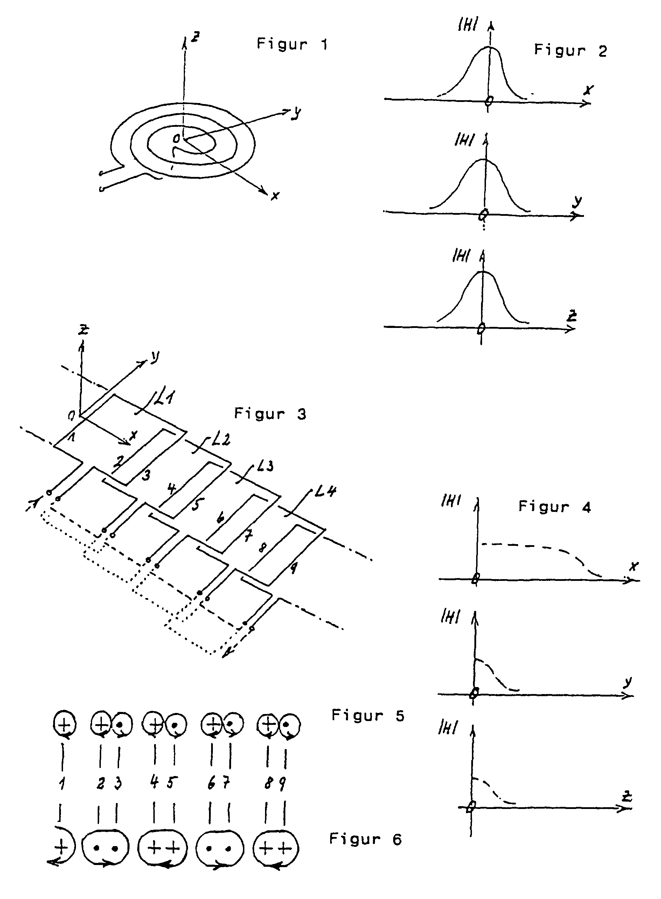

In der unter 0239098 A2 veröffentlichten Europäischen Patentanmeldung wird eine zur Erzeugung eines flächenhaften Magnetfeldes unter anderem (Figur 2) eine Spulenmatte mit einer einzigen Leiterschleife dargestellt und beschrieben, deren Leiter mäanderförmig geführt ist. Damit wird zwar im Vergleich zu verteilten Spiralspulen in einem gewissen Bereich ein gleichförmigeres Magnetfeld erreicht. Die Gestaltung der Stärke des Magnetfeldes über die Mattenfläche ist jedoch eingeschränkt und im Bereich der Zuleitung treten nach wie vor, im Gegensatz zu einer erfindungsgemässen Ausgestaltung starke und unerwünschte Abstrahlungen in die Umgebung auf.

Nachstehend wird die Erfindung anhand einer Zeichnung, die Ausführungsbeispiele wiedergibt, näher erläutert.

In dieser Zeichnung zeigt
- die Figur 1: ein Spulenmatten-Element, das als Leiterspirale ausgebildet ist,
- die Figur 2: den Feldstärkeverlauf des Magnetfeldes der Leiterspirale in Bezug auf das Koordinatensystem in der Figur 1,
- die Figur 3: ein Spulenmatten-Element aus vier, gegeneinander versetten Leiterschleifen,
- die Figur 4: den Feldstärkeverlauf des Magnetfeldes einer Anordnung nach der Figur 3 in Bezug auf deren Koordinatenssystem,
- die Figur 5: schematisch den Verlauf der magnetischen Feldlinien einer Anordnung nach der Figur 3 bei gleichsinniger Speisung der einzelnen Leiterschleifen,
- die Figur 6: schematisch den Verlauf der magnetischen Feldlinien einer Anordnung nach der Figur 3 bei gegensinniger Speisung der einzelnen Leiterschleifen,
- die Figur 7: ein Spulenmatten-Element aus zwei länglichen Leiterschleifen,
- die Figur 8: eine Speisungsvariante für eine Anordnung nach der Figur 7,
- die Figur 9: das Blockschaltbild einer Generatorschaltung für eine Speisung entsprechend der Figur 8,
- die Figur 10: ein Bild zur Erläuterung der Ableitung einer besonderen Ausführungsform eines Spulenmatten-Elements
- die Figur 11: ein weiteres Bild zur Erläuterung der Ableitung der besonderen Ausführungsform eines Spulenmatten-Elements
- die Figur 12: eine besondere Ausführungsform einer Spulenmatte
- die Figur 13: die Verteilung des Betrags des magnetischen Feldsstärke einer Spulenmatte nach der Figur 12 in Bezug auf die Koordinate x ihres Koordinatensystems,
- die Figur 14: die Verteilung des Betrags des magnetischen Feldsstärke einer Spulenmatte nach der Figur 12 in Bezug auf die Koordinate x ihres Koordinatensystems, bei einer Verminderung des Einzelleiterabstands an den Mattenrändern,
- die Figur 15: eine konstruktive Amtührung einer Spulenmatte und
- die Figur 16: eine Anordnung von Spulenmatten zur Erzeugung eines wirbelnden Magnetfeldes in einem vorgegebenen Raumbereich.

In der Figur 1 ist in Schrägansicht eine als Spirale ausgebildete Spule Sp gezeigt, die von einem nicht dargestellten Generator mit Strom gespeist wird. In der Schrägansicht ist ein karthesisches Koordinatensytem eingezeichnet, dessen Nullpunkt 0 im Zentrumm der Spirale liegt. Diese Spulenausbildung hat wegen der Tatsache, daß alle magnetischen Feldlinien durch das Zentrum hindurchgehen zur Folge, daß sich ein Verlauf des Betrags der magnetischen Feldstärke ergibt, der in etwa dem einer Glokkenkurve entspricht. Dieser Verlauf des Betrags, bzw. Absolutwertes ¦H¦ ist in der Figur 2 für die drei Koordinatenrichtungen x, y und z schematisch gezeigt.

Die Folge ist die einleitend erwähnte, in vielen Fällen störende Feldstärkespitze im Zentrumsbereich der Spirale. Die Darstellung der Verläufe ist dabei nur als Hüllkurve zu verstehen und läßt die von der Anzahl der Windungen abhängige Wellung der Verläufe außer Betracht.

Bei dem in der Figur 3 ebenfalls in Schrägansicht dargestellten Ausführungsbeispiel der Erfindung werden demgegenüber mehrerer Leiterschleifen, beispielsweise die vier Leiterschleifen L1, L2, L3 und L4, in Richtung der Koordinate x gegeneinander versetzt und sich teilweise überlappend angeordnet. Die einzelnen Leiterschleifen haben in etwa die gleiche Schleifenfläche. Durch eine solche Anordnung und Ausbildung ergibt sich ein Verlauf des Betrags der magnetischen Feldstärke in Richtung der drei Koordinaten x, y und z wie er in der Figur 4 schematisch dargestellt ist. Auch hier sind nur die Hüllkurven dargestellt. Die Maxima der drei Verläufe sind wesentlich abgesenkt und in Richtung der Korrdinate x ist das magnetische Feld bis weit nach außen relativ gleichförmig und dann erst am Ende der Reihe von Leiterschleifen auf den Wert von praktisch Null abfallend.

Eine solche Reihe von Leiterschleifen kann, so wie in der Figur 3 durch gestrichelte Verbindungen gezeigt in der Weise in Serie geschaltet werden, daß sie - von oben gesehen - alle im gleichen Richtungssinn vom Betriebsstrom durchflossen werden. Dieser Betriebsfall ist in der Figur 5 für eine Ebene dargestellt, die durch die Koordinaten x und z bestimmt ist. In die Zeichenebene eintretende Ströme sind als Kreuze und aus der Zeichenebene heraustretende Ströme als Punkte dargestellt. Die in der Figur 3 mit den Ziffern 1 bis 9 gekennzeichneten Leiter der einzelnen Leiterschleifen entsprechen den mit den gleichen Ziffern gekennzeichneten Leitern in der Figur 5. Die magnetischen Feldlinien, welche die einzelnen Leiter umschließen, haben in Richtung der Koordinate x alternierend wechselnde Richtung. Der Grad der Überlappung und die Breite der einzelnen Leiterschleifen in Richtung der x-Koordinate bestimmt den Detailverlauf der magnetischen Feldstärke, und zwar nach Größe und Richtung.

Durch eine Serienschaltung der einzelnen Leiterschleifen in dem Sinn, daß benachbarte Leiterschleifen jeweils gegensinnig von Betriebstrom durchflossen werden, ergibt sich das StromfluBbild nach der Figur 6. Die entsprechende elektrische Verbindung der einzelnen Leiterschleifen ist in der Figur 3 durch gepunktete Verbindungen dargestellt. Wie aus den schematisch eingezeichneten magnetischen Feldlinien erkennbar, wird durch diese Speisung die zur Richtung der x-Koordinate parallele Feldkomponente wesentlich stärker ausgeprägt.

In beiden Fällen ist die aus der Figur 2 ersichtliche Feldstärkenspitze weitgehend vermindert.

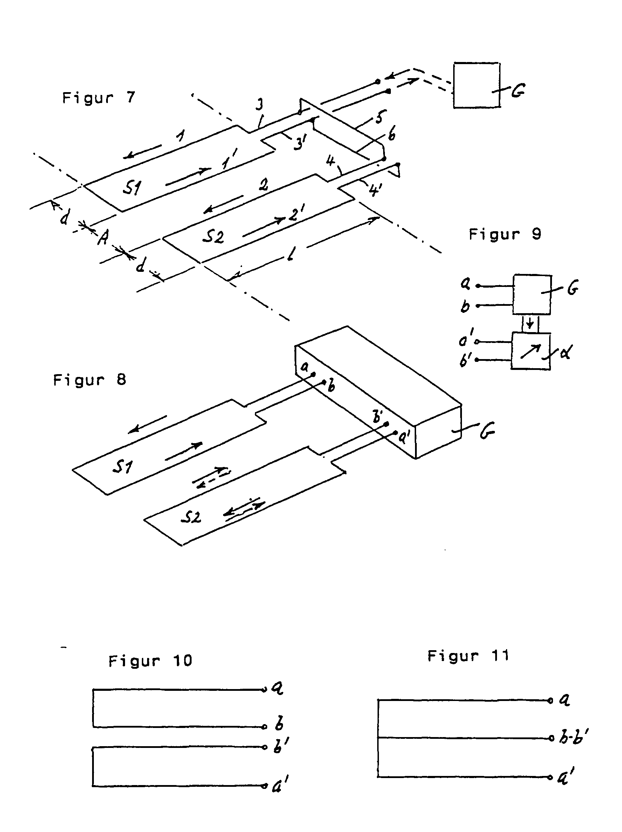

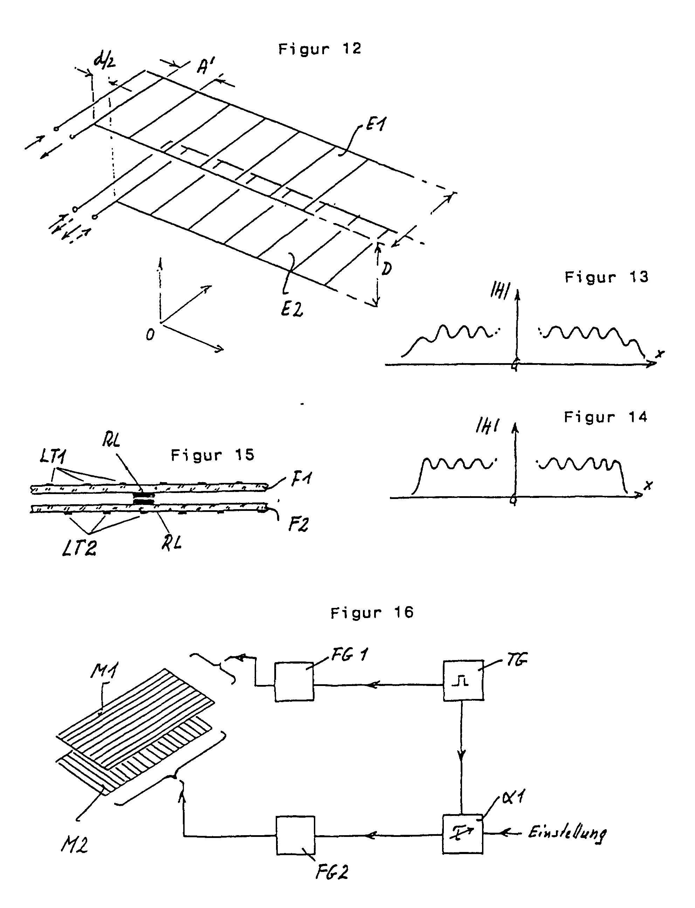

Anhand der Figuren 7 und 8 werden zunächst die Feldverhältnisse behandelt, die sich bei zwei, sich nicht überlappenden Leiterschleifen ergeben, um das Verständnis einer anhand der Figur 12 erläuterten erfindungsgemässen Ausführungsform zu erleichtern. Eine Untersuchung zeigt, daß im wesentlichen die gleichen Bedingungen gegeben sind, wie bei dem Ausführungsbeispiel nach der Figur 3.

In der Figur 7 sind zwei längliche Leiterschleifen S1 und S2 zumindest angenähert in einer Fläche, welche durch die von der Spulenmatte eingenommene Fläche bestimmt wird, nebeneinander angeordnet. Die Leiterschleife S1 enthält die Längsleiter 1 und 1' und die Leiterschleife S2 die Längsleiter 2 und 2'. Die Leiterschleife S1 ist über die Zuleitungen 3 und 3' mit einem, einen gewünschten zeitlichen Verlauf seiner Amplitude aufweisenden Speisungsstrom erzeugenden, elektrischen Generator G verbunden. Gleichartig ist die Leiterschleife S2 über die Zuleitungen 4 und 4' mit dem Generator G verbunden.

Zur Reduzierung der zum Generator G führenden Leitungen sind die Leiterschleifen S1 und S2 über Leiterbügel 5 und 6 parallel geschaltet.

Bei dieser Schaltung ergibt sich in einer senkrecht die Leiter schneidenden Ebene ein Verlauf des Magnetfeldes, der dem nach der Figur 5 schematisch entspricht. Sind die Leiter 1' und 2 eng benachbart, so ist in ihrem Bereich eine Anhebung der Stärke des Magnetfeldes gegenüber den Bereichen um die Leiter 1 und 2' gegeben. Durch Vergrößerung des seitlichen Abstandes A der Leiter 1' und 2 läßt sich die Anhebung in gewissen Grenzen absenken.

Verbindet man mit dem Generator über die Leiterbügel 5 und 6 - gegensinnig zur Darstellung in der Figur 7 - die Zuleitungen 3 und 4 bzw. die Zuleitungen 3' und 4', so ergibt sich ein Verlauf des Magnetfeldes der in etwa dem nach der Figur 6 entspricht. Die Felder um die Leiter 1' und 2 kompensieren sich bei einem geringen seitlichen Abstand A dieser Leiter teilweise, wodurch im Bereich dieser Leiter die Stärke des Magnetfeldes abgesenkt wird. Durch eine Vergrößerung des seitlichen Abstandes A der Leiter 1' und 2 kann diese Absenkung reduziert werden. Eine Vergrößerung der Breite d der einzelnen Leiterschleife bewirkt ein Anhebung der magnetischen Feldstärke in Richtung der Koordinate z (vergl. Figur 3). Die Schleifenlänge 1 bestimmt den Längenbereich über den wenigstens nahezu gleiche Feldverhältnisse gegeben sind.

In der Figur 8 ist eine Schaltung gezeigt, bei der die beiden Leiterschleifen S1 und S2 über ihre Anschlüsse a und b bzw. a' und b' getrennt von einem, zwei Ausgänge aufweisenden Generator G gespeist werden. Zwischen den Strömen, die von den beiden Ausgängen in die Leiterschleifen S1 und S2 eingespeist werden, ist eine Phasenverschiebung α einstellbar. Erreicht werden kein dies beispielsweise dadurch, daß - wie in der Figur 9 schaltungstechnisch dargestellt - vom Generator G der eine Ausgang, zum Beispiel a, b, unmittelbar gespeist wird, wahrend der andere Ausgang über ein vorzugsweise einstellbares Phasendrehglied α mit dem Generator G verbunden ist. Wird eine Phasenverschiebung von 180° gewünscht, entsprechend einer bevorzugten Ausführungsform, so kann das Phasendrehglied entweder ein Phasenumkehr-Transformator oder eine Phasenumkehrschaltung an sich bekannter Art sein. Ein Transformator empfiehlt sich vor allem dann, wenn der Speisestrom der Leiterschleifen frei von einer Gleichstrom-Komponente ist. Enthält hingegen der Speisestrom eine Gleichstrom-Komponente oder über längere Zeit quasi eine solche, so empfiehlt sich die Verwendung einer der bekannten Phasenumkehrschaltungen auf Halbleiter-Basis.

In der Figur 8 sind als stark ausgesogene Pfeile die Stromrichtungen in den beiden Leiterschleifen S1 und S' für den Fall einer Phasenverschiebung von 0° eingezeichnet. Führt man die erwähnte Phasenverschiebung α von 180° für den Anschluß a', b' gegenüber dem Anschluß a, b ein, so ergibt sich in der Leiterschleife die durch gestrichelte Pfeile kenntlich gemachte Stromrichtung. Für diesen Fall heben sich die von den beiden Leitern b und b' ausgehenden Magnetfelder bei geringem gegenseitigen Abstand d der beiden Leiter praktisch gegenseitig weitgehend auf. Es entspricht dies dem Ersatzschaltbild nach der Figur 10. Ersetzt mm die beiden Leiter b und b' - wie in dem Ersatzschaltbild der Figur 11 gezeigt - durch einen gemeinsamen Leiter b-b', b-b', bezogen auf einen Augenblickswert, so kompensieren sich für den Fall der Phasenverschiebung von α = 180° die Stromflüsse in dem gemeinsamen Leiter b-b'. Sind die beiden Stromflüsse amplitudengleich, so tritt eine vollständige Kompensation ein. Das bedeutet, daß der gemeinsame Leiter b-b' stromlos ist und kein Magnetfeld erzeugt. Durch eine solche Ausbildung kann man also erreichen, daß die Leiterrückführung der beiden Leiterschleifen S1 und S2 im Gegensatz zu der Stromrückführung bei der erwähnten Flachspirale kein unerwünschtes Magnetfeld erzeugt.

Diese Ausführung eröffnet eine besonders vorteilhafte Ausbildung für eine Spulenmatte. Ersetzt man die Leiter a und a' durch eine Vielzahl paralleler Einzelleiter, so wie es in der Figur 12 schematisch gezeigt ist - und verbindet diese in der dargestellten Weise miteinander, so erhält man zwei übereinander liegende Leiterebenen E1 und B2. Durch einen seitlichen Versatz, vorzugsweise um den halben seitlichen Leiterabstand d und eine geringen Abstand D der beiden Leiterebenen entsteht eine Vielzahl von Leiterschleifen, die sich durch einen hohen Grad von Gleichförmigkeit des im Speisungsfall erzeugten Gesamt-Magnetfeldes auszeichnen, und zwar über die gesamte, durch die Leiterebenen bestimmte Fläche. Auch hierbei können die beiden Rückleiter b und b' zu einem gemeinsamen Rückleiter vereinigt werden. Im Fall von getrennten Rückleitern werden diese zweckmäßig möglichst eng benachbart zueinander geführt, um bei gegenphasig in ihnen fließenden Strömen eine möglichst vollständige Feldkompensation der von ihnen ausgehenden Magnetfelder zu erzwingen. Die Rückleiter beziehungsweise der gemeinsame Rückleiter können, wie dargestellt, an einem Seitenrand der Spulenmatte angeordnet werden. Es ist jedoch auch möglich diese Leiter innerhalb der Spulenmatte vorzusehen.

Wählt man den gegenseitigen seitlichen Abstand A' der einzelnen Leiter in der einzelnen Leiterebene gleich groß, so ergibt sich eine Verteilung des Magnetfeldes in einer senkrecht die Leiterebene schneidenden Ebene wie es in der Figur 13 schematisch skizziert ist. Auf der Abszisse ist der seitliche Abstand x von der Mitte der Leiterebene aufgetragen. Auf der Ordinate ist der Betrag ¦H¦ der magnetischen Feldstärke H aufgetragen. Lediglich an den seitlichen Rändern der Leiterebenen fällt der Wert des Betrags ab. Wenn das im Einzelfall unerwünscht ist, so kann dem dadurch begegnet werden, daß der gegenseitige seitliche Leiterabstand d in den Randbereichen gegenüber dem Wert im Mittelbereich der Leiterebenen vermindert wird. Man erhält dann eine Feldverteilung, die in etwa dem in der Figur 14 skizzierten Verlauf entspricht. Die Verläufe entsprechen in in etwa einer Chebycheff-Kurve. Die Anzahl der Maxima entspricht der Anzahl der Leiter und der Betragsunterschied zwischen den Maxima und den Minima wird durch den gegenseitigen seitlichen Abstand d der einzelnen Leiter festgelegt.

Man kann auch die Ströme in den einzelnen Leitern im Betrag unterschiedlich machen, beispielsweise durch entsprechend unterschiedliche Widerstandswerte der einzelnen Leiter (z.B. unterschiedliche Querschnitte und/oder unterschiedliche spezifische Widerstandswerte aufweisende Materialien).

Da die erfindungsgemäße Ausgestaltung hinsichtlich der Länge 1 der einzelnen Leiter der Leiterebenen eine weitgehende Freiheit ergibt, ist es möglich nicht nur über kleinere Flächen, wie die eines Kissens, ein weitgehend gleichförmiges Magnetfeld sicherzustellen, sondern auch über größere Flächen, die eine größere Länge als Breite haben. Ein Beispiel ist die erwähnte Behandlungsdecke.

In der Figur 15 ist in einem Schnittbild eine konstruktive Ausgestaltung einer Spulenmatte wiedergegeben. Der Schnitt verläuft dabei in einer die einzelnen Leiter Lt1 bzw. Lt2 schneidenden, senkrecht zu der durch die Koordinaten x und y in der Figur 12 festgelegten Ebene. Auf zwei dünnen, vorzugsweise biegsamen Folien F1 und F2 aus elektrisch isolierendem Material sind nach Art einer "Gedruckten Schaltung" die einzelnen Leiter L aufgebracht. Jede der beiden Folien F1 bzw. F2 ist in gleicher Weise mit einem Rückleiter RL versehen. Die Folien haben eine Länge und Breite die den gewünschten Abmessungen der Spulenmatte entspricht. An ihren Enden sind die einzelnen Leiter Lt1 bzw. Lt2 entsprechend der Figur 12 elektrisch geschaltet. Die Folien können auch so zueinander angeordnet werden, daß ihre von den Leitern Lt1 oder Lt2 freien Flächen einander zugewandt sind, oder daß eine der Folien mit ihrer Leiterseite (Lt1 oder Lt2) auf der Rückleiterseite der anderen Folie anliegt.

Eine Abwandlung ist dahingehend möglich, daß nur eine Folie aus elektrisch isolierendem biegsamen Material vorgesehen wird und die Leiter Lt1 bzw Lt2 der beiden Leiterebenen auf den gegenüberliegenden Außenflächen dieser Folie aufgebracht werden. Für diesen Fall empfiehlt es sich, die beiden oder einen gemeinsamen Rückleiter an einem Seitenrand der Folie anzuordnen.

Zur Verbindung der einzelnen Leiter L untereinander und/oder mit den(dem) Rückleiter(n) können die für gedruckte Schaltungen bekannten Methoden, wie Durchkontaktierungen, Mehrschicht-Ausbildung und Leiterschleifen Anwendung finden.

Es ist auch möglich die einzelnen Leiter als hochelastische, biegsame Leiter auszubilden und in eine Textilbahn in an sich bekannter Weise einzubringen. Diese Technik ist beispielsweise durch elektrische Heizkissen und Heizdecken bekannt. Diese Ausbildung kann vor allem deshalb ohne Bedenken Anwendung finden, weil die elektrischen Potentialunterschiede zwischen den einzelnen Leitern sehr gering sind. Das bedingt die für die Magnetfeld-Erzeugung nötige Stromspeisung der einzelnen Leiter, die eine geringen elektrischen Widerstand des einzelnen Leiters erfordert. Soll beispielsweise in einem Leiter ein Strom von 1 Ampere fließen und hat der Leiter einen elektrischen Widerstand von 1 Ohm, so beträgt der Spannungsabfal über seine gesamte Länge nur 1 Volt - ein Wert der weit unter der sogenannten Gefährdungsgrenze liegt -.

An sich könnte im Fall eines gemeinsamen Rückleiters und im Idealfall sich auf den Wert Null kompensierender Rückströme, der gemeinsame Rückleiter entfallen. In der Praxis ist es jedoch empfahlenswert den gemeinsamen Rückleiter zu belassen um eine Potentialfixierung sicherzustellen. Der Leiterquerschnitt und die Belastbarkeit der Rückleiter sollte so groß gewählt werden, daß er mit der Summe der Ströme der einzelnen Leiter einer der beiden Leiterebenen belastbar ist. Diese Belastung kann nämlich dann auftreten, wenn im Betriebsfall durch einen. Ausfall der elektrischen Speisung eine der beiden Leiterebenen ausfällt oder unterschiedlich wird.

Durch die Erfindung wird noch eine weitere wesentliche Möglichkeit eröffnet. Ordnet man - so wie in der Figur 16 schematisch dargestellt - zwei Spulenmatten M1, M2 sich kreuzend und in geringem gegenseitigen Abstand übereinander an, so ist bei Speisung beider Spulenmatten das resultierende Magnetfeld die vektorielle Summe aus den beiden Einzelfeldern. Speist man die untere Spulenmatte aus einem ersten Generator und die obere Spulenmatte aus einem weiteren, gleichartigen Generator, so kann man durch eine Phasenverschiebung zwischen den Ausgangsströmen der beiden Generatoren den vektoriellen Summanvektor in seiner räumlichen Ausrichtung beeinflussen. Dies ist zum Beispiel dadurch möglich, daß der zweite Generator von dem ersten Generator über ein Phasendrehglied entsprechend-der Figur 9 gesteuert wird.

In Weiterbildung der Erfindung sind die beiden Generatoren, wie in der Figur 16 schematisch dargestellt, als sogenannte Funktionsgeneratoren FG1 und FG2 ausgebildet, die ein formgleiches Ausgangssignal liefern. Solche Funktionsgeneratoren sind an sich allgemein bekannt und beispielsweise in der einleitend genannten Internationalen Patentanmeldung nebst einer Literaturangabe beschrieben. Die Funktionsgeneratoren sind des weiteren in an sich bekannter Weine so ausgebildet, daß sie durch ein Triggersignal zur Abgebe beispielsweise einer oder auch mehrerer vollständiger Perioden des Ausgangssignals auslösbar sind. Durch eine Schaltung TG (Triggergenerator) und α1 (digitale Laufzeitkette) werden zwei gegenseitig einstellbare phasenverschobene Triggersignalfolgen für das zeit- bzw. phasenversetzte Arbeiten der beiden Funktionsgeneratoren erzeugt.

Dadurch wird die Möglichkeit eröffnet die Phasenverschiebung der Ströme in den einzelnen Leitermatten, beispielsweise durch die einstellbare Laufzeitkette α1 in gewünschter Weise zwischen den Werten 0 und 2π, vorzugsweise periodisch zu verändern. Bei einer solchen Betriebsart rotiert der Summenvektor sozusagen um die z-Koordinate und erzeugt mit anderen Worten unterhalb und oberhalb der beiden Spulenmatten ein wirbelndes Magnetfeld.

Im Vorstehenden wurde gezeigt, daß es möglich ist die einzelnen Leiterschleifen elektrisch in Serie oder parallel zu betreiben. Im Einzelfall ist die Betriebsart abhängig von der Gesamt-Eingangsimpedanz, die an den Anschlüssen der einzelnen Spulenmatte gewünscht wird. Da die Stärke des erzeugten Magnetfeldes primär von der Stärke des Stromes in den einzelnen Leitern abhängig ist, ist eine vorherrschende Stromspeisung der einzelnen Leiterschleifen anzustreben. Dies erfordert einem niedrigen Innenwiderstandswert des speisenden Generators. Aus Anpassungsgründen empfiehlt es sich deshalb die Anschlußimpedanz der einzelnen Spulenmatte entsprechend anzupassen. Um Verzerrungen des Stromflusses in den Leiterschleifen zu vermeiden ist es desweiteren empfehlenswert, Resonanzen im Bereich der Spulenmatten weitgehend zu vermeiden, indem man diese durch ohmsche Widerstände aperiodisch macht oder als an den Generator angepaßten Tiefpaß ausbildet, dessen obere Grenzfrequenz der höchsten vom Generator abzugebenden Frequenz Rechnung trägt. Solche Widerstände können durch die einzelnen Leiter selbst gebildet werden, indem ihr Leiterquerschnitt und/oder das für sie verwendete Leitermaterial entsprechend gewählt werden.

Durch die Länge 1 der einzelnen Leiterschleife, deren Breite d und die Anzahl der nebeneinander angeordneten und die Lage der Leiterschleifen zueinander - soweit der Schleifenabstand A, wie im Fall der Figur 12 gleich Null ist - hat man die Möglichkeit auch über große Flächen ein gleichförmiges Magnetfeld zu erzeugen. So ist es möglich die Länge 1 der einzelnen Leiterschleife der Länge einer Behandlungsdecke nahezu gleich zu machen und so viele Leiterschleifen nebeneinander, gegebenenfalls sich teilweise überlappend, anzuordnen, daß nahezu die gesamte Breite der Behandlungsdecke abgedeckt wird. Die Leiter der einzelnen Leiterschleifen müssen auch nicht zwingend gerade geführt sein, sondern können beispielsweise nach Art einer Schlangenlinie vorgesehen werden. Auch ist es möglich über die Länge der einzelnen Leiterschleife den Abstand ihrer Leiter unterschiedlich zu machen, beispielsweise für den Fall, daß für einzelne Bereiche der Decke unterschiedliche Intensitäten des Magnetfeldes gefordert werden.

Wesentlich für eine Ausführung entsprechend der Figur 12 ist, daß bei einer Speisung von E2 in gestrichelt eingezeichneten Sinn die einzelnen Leiter L von E1 und E2, auf den Augenblickswert bezogen, im gleichen Richtungssinn stromdurchflossen sind. Dadurch entsteht eine einheitliche Ausrichtung der magnetischen Feldlinien über die Gesamtbreite von E1, E2. Erreichbar ist diese Speisung durch einen Phasenunterschied von 180° der in E1 und E2 eingespeisten Ströme. Die Ströme in einem gemeinsamen Rückleiter kompensieren sich dabei. Diese Speisungsart ist auch für jede der beiden Spulenmatten M1, M2 in Figur 16 mit Vorteil einsetzbar.

## Patentansprüche

1. Spulenmatte zur Erzeugung eines flächenhaften Magnetfeldes, bei der mehrere, über eine Anschlußleitung mit einem Generator zur Stromspeisung verbindbare, nebeneinander über die Mattenfläche verteilte, in Serien- und/oder in Parallelschaltung elektrisch verbundene Einzelspulen vorgesehen sind, **dadurch gekennzeichnet, daß** die Einzelspulen als untereinander wenigstens nahezu flächeninhaltsgleiche Leiterschleifen ausgebildet sind, die zumindest angenähert Rechteckform haben, und daß die zumindest angenähert Rechtform habenden Leiterschleifen sich teilweise überlappend und mit ihren entsprechenden Rechteckseiten zueinander parallel über die Mattenfläche weitgehend gleichförmig verteilt angeordnet sind.

2. Spulenmatte nach Anspruch 1, **dadurch gekennzeichnet, daß** die zumindest nahezu Rechteckform habenden Leiterschleifen von länglicher Form sind.

3. Spulenmatte nach Anspruch 1 oder 2, **dadurch gekennzeichnet, daß** zumindest für einen Teil der Leiterschleifen eine Verbindungsschaltung zur Anschlußleitung mit derartiger Ausbildung vorgesehen ist, dass räumlich benachbarte Leiter vom Speisungsstrom in der gleichen Richtung durchflossen werden.

4. Spulenmatte nach Anspruch 1 oder 2, **dadurch gekennzeichnet, daß** zumindest für einen Teil der Leiterschleifen eine Verbindungsschaltung zur Anschlußleitung mit derartiger Ausbildung vorgesehen ist, dass räumlich benachbarte Leiter vom Speisungsstrom in gegensinniger Richtung durchflossen werden.

5. Spulenmatte nach einem der Ansprüche 1 bsi 4, **dadurch gekennzeichnet, daß** zumindest ein Leiterabschnitt einer Leiterschleife aus einer Mehrzahl getrennter, elektrisch jedoch parallel geschalteter, eine Leitermatte bildender Leiter besteht, die unter Bildung sich überlappender Leiterschleifen in der Mattenfläche nebeneinander liegend angeordnet sind.

6. Spulenmatte nach Anspruch 5, **dadurch gekennzeichnet, daß** zwei derart ausgebildete Leitermatten in der Spulenmatte, hinsichtlich der Leiter seitlich versetzt, übereinanderliegend vorgesehen sind.

7. Spulenmatte nach Anspruch 6, **dadurch gekennzeichnet, daß** die Rückleiter beider Leitermatten zueinander eng benachbart angeordnet sind.

8. Spulenmatte nach einem der Ansprüche 5 bis 7, **dadurch gekennzeichnet, daß** der Leitungsquerschnitt des Rückleiters der einzelnen Leitermatte dem Gesamtquerschnitt der elektrisch parallelen Leiter der jeweiligen Leitermatte entspricht.

9. Spulenmatte nach einem der Ansprüche 6 bis 8, **dadurch gekennzeichnet, daß** die beiden Leitermatten einen gemeinsamen Rückleiter haben.

10. Spulenmatte nach einem der vorhergehenden Ansprüche, **dadurch gekennzeichnet, daß** Hin- und Rückleiter der einzelnen Leiterschleife wenigstens nahezu die gleiche Anzahl von Einzelleitern haben.

11. Spulenmatte nach einem der Ansprüche 6 bis 10, **dadurch gekennzeichnet, daß** die beiden Leitermatten wenigstens nahezu gleich ausgebildet sind, und daß der seitliche Versatz der beiden Leitermatten zumindest angenähert dem halben Leiterabstand in einer der beiden Leitermatten entspricht.

12. Spulenmatte nach einem der Ansprüche 6 bis 11, **dadurch gekennzeichnet, daß** der Leiterabstand in Bereichen, in denen eine Anhebung oder Absenkung des Magnetfeldes gefordert ist, derart unterschiedlich gegenüber den anderen Bereichen ausgebildet ist, daß sich der für diesen Bereich geforderte Unterschied der magnetischen Feldstärke in Bezug auf die anderen Bereiche einstellt.

13. Spulenmatte nach Anspruch 12, **dadurch gekennzeichnet, daß** der Leiterabstand im seitlichen Endbereich der einzelnen Leitermatte derart verringert ist, daß der ansonsten dort im Betriebsfall erfolgende Abfall der magnetischen Feldstärke wenigstens nahezu ausgeglichen ist.

14. Spulenmatte nach einem der Ansprüche 6 bis 13, **dadurch gekennzeichnet, daß** die einzelnen Leiter in Bereichen, für die eine Anhebung oder Absenkung des Magnetfeldes gefordert ist, derart im Querschnitt und/oder Leitermaterial unterschiedlich ausgebildet ist, daß sich der für diesen Bereich geforderte Unterschied der magnetischen Feldstärke in Bezug auf die anderen Bereiche im Betriebsfall einstellt.

15. Spulenmatte nach einem der Ansprüche 6 bis 14, **dadurch gekennzeichnet, daß** die Speisungsschaltung zur Verbindung der Leiter mit der Anschlußleitung derart ausgebildet ist, daß sich die Ströme in den Rückleitern der Spulenmatte zumindest hinsichtlich des von ihnen ausgehenden Magnetfeldes kompensieren.

16. Erweiterte Spulenmatte aus zwei Spulenmatten nach einem der vorhergehengehenden Ansprüche, **dadurch gekennzeichnet, daß** die zwei Spulenmatten sich kreuzend und in geringem gegenseitigen Abstand aufeinanderliegend vorgesehen sind, und daß jede der beiden Spulenmatten mit einem eine gegenseitig unabhängige Speisung sicherstellenden Anschluß versehen ist.

17. Gerät zur Erzeugung eines flächenhaften Magnetfeldes mit einer erweiterten Spulenmatte nach Anspruch 16 sowie einem Generator zur Speisung der Spulenmatte, bei dem der Generator zwei Ausgänge zur getrennten Speisung der beiden Spulenmatten hat.

18. Gerät nach Anspruch 17, **dadurch gekennzeichnet, daß** der Generator mit einer Schaltung zur Erzeugung eines Speisungsstromes vorgegebenen zeitlichen Amplitudenverlaufs versehen ist, die den einen der beiden Ausgänge speist, und daß der andere Ausgang über ein Phasenumkehrglied mit der Schaltung zur Erzeugung des Speisungsstromes verbunden ist.

19. Gerät nach Anspruch 17, **dadurch gekennzeichnet, daß** zwei, ein formgleiches Ausgangssignal liefernde, Einzelgeneratoren vorgesehen sind, deren jeder einen der beiden Ausgänge speist, und daß die Phasenlage zwischen den Ausgangssignalen beider Einzelgeneratoren veränderbar ist.

20. Gerät nach Anspruch 19, **dadurch gekennzeichnet, daß** die Einzelgeneratoren als hinsichlich des Startzeitpunkts ihres Ausgangssignals triggerbare Funktionsgeneratoren ausgebildet sind, und daß eine Schaltung zur, vorzugsweise einstellbaren Abgabe von gegeneinander zeitverzögerten von Triggersignalen für die beiden Einzelgeneratoren vorgesehen ist.

## Claims

1. Coil mat for the generation of two-dimensional magnetic fields, in the said mat of which there are a number of single coils, which can be connected via a connecting lead to a generator for supplying current, which are distributed adjacently over the mat area and which are electrically connected in series and/or parallel, **characterised in that** the single coils are configured amongst themselves as conductor loops covering at least almost the same area, the said loops covering at least approximately a rectangular shape, and that the conductor loops having at least an approximate rectangular shape partially overlap and are arranged distributed essentially uniformly over the mat surface with their appropriate rectangle sides parallel to one another.

2. Coil mat according to Claim 1, **characterised in that** the conductor loops having at least approximately a rectangular shape are longitudinal in shape.

3. Coil mat according to Claim 1 or 2, **characterised in that** at least for one part of the conductor loops a connection circuit to the connecting lead is present with such a configuration that spatially adjacent conductors have supply current flowing through them in the same direction.

4. Coil mat according to Claim 1 or 2, **characterised in that** at least for one part of the conductor loops a connection circuit to the connecting lead is present with such a configuration that spatially adjacent conductors have supply current flowing through them in opposing directions.

5. Coil mat according to Claims 1 to 4, **characterised in that** at least one section of conductors of a conductor loop consists of a number of separate, electrically switched however in parallel, conductors which form a conductor mat and which are arranged lying adjacently to form overlapping conductor loops in the mat area.

6. Coil mat according to Claim 5, **characterised in that** two conductor mats so formed in the coil mat are provided with a sideways offset and are overlapping with respect to the conductors.

7. Coil mat according to Claim 6, **characterised in that** the return conductors of both conductor mats are arranged closely adjacent to one another.

8. Coil mat according to one of the Claims 5 to 7, **characterised in that** the conductor cross-sectional area of the return conductor of the individual conductor mats corresponds to the total cross-sectional area of the electrically parallel conductors of the respective conductor mat.

9. Coil mat according to one of the Claims 6 to 8, **characterised in that** the two conductor mats have a common return conductor.

10. Coil mat according to one of the previous claims, **characterised in that** the feed and return conductors of the individual conductor loops have at least almost the same number of single conductors.

11. Coil mat according to one of the Claims 6 to 10, **characterised in that** the two conductor mats are at least formed almost the same, and that the sideways offset of the two conductor mats corresponds at least approximately to half the conductor spacing in one of the two conductor mats.

12. Coil mat according to one of the Claims 6 to 11, **characterised in that** the conductor spacing in regions, in which an increase or reduction in the magnetic field is demanded, is formed differently compared to the other regions such that the difference in the magnetic field strength demanded for this region is set in relation to the other regions.

13. Coil mat according to Claim 12, **characterised in that** the conductor spacing in the sideways end region of the individual conductor mat is reduced such that the drop in the magnetic field strength otherwise occurring there in operation is at least almost compensated.

14. Coil mat according to one of the Claims 6 to 13, **characterised in that** the individual conductors in the regions for which an increase or reduction of the magnetic field is demanded is formed differently in cross-section and/or conductor material such that the difference in the magnetic field strength demanded for this region is set in relation to the other regions in operation.

15. Coil mat according to one of the Claims 6 to 14, **characterised in that** the feed circuit for connection of the conductors with the connecting lead is formed such that the currents in the return conductors of the coil mat at least compensate with regard to the magnetic fields emanating from them.

16. Extended coil mat of two coil mats according to one of the previous claims, **characterised in that** the two coil mats cross and are provided lying overlapping one above the other at a slight mutual distance and that each of the two coil mats is provided with a connection ensuring a mutually independent supply.

17. Device for the generation of a two-dimensional magnetic field with an extended coil mat according to Claim 16 and a generator for the supply of the coil mat, in which the generator has two outputs for the separate supply of the two coil mats.

18. Device according to Claim 17, **characterised in that** the generator is provided with a circuit for the generation of a supply current with a specified progression of amplitude in time, which supplies one of the two outputs and that the other output is connected via a phase reversal unit to the circuit for the generation of the supply current.

19. Device according to Claim 17, **characterised in that two** single generators supplying identically shaped output signals are provided, each of which supplies one of the two outputs and that the phase angle between the output signals of both individual generators can be changed.

20. Device according to Claim 19, **characterised in that** the individual generators are formed as triggerable function generators with regard to the starting time point of their output signal and that a circuit for the preferably adjustable output of mutually time delayed trigger signals is provided for both individual generators.

## Revendications

1. Mat de bobines destiné à générer un champ magnétique plan, pour lequel sont prévues plusieurs bobines individuelles pouvant être reliées à un générateur par une ligne de branchement afin d'être alimentées en courant, réparties les unes à côtés des autres sur la surface du mat, couplées électriquement en série et/ou en parallèle, **caractérisé en ce que** les bobines individuelles sont composées de spires de conducteur ayant au moins presque la même superficie et une forme au moins approximativement rectangulaire, et **en ce que** les spires de conducteur ayant une forme au moins approximativement rectangulaire se chevauchent partiellement et sont réparties sur la surface du mat de façon à peu près homogène, leurs côtés de même longueur respectifs étant parallèles entre eux.

2. Mat de bobines selon la revendication 1, **caractérisé en ce que** les spires de conducteur ayant une forme au moins approximativement rectangulaire sont de forme allongée.

3. Mat de bobines selon la revendication 1 ou 2, **caractérisé en ce qu'**un circuit de connexion à la ligne de raccordement est prévu au moins pour une partie des spires de conducteur et conçu de telle sorte que le courant d'alimentation parcourt les conducteurs voisins dans le même sens.

4. Mat de bobines selon la revendication 1 ou 2, **caractérisé en ce qu'**un circuit de connexion à la ligne de raccordement est prévu au moins pour une partie des spires de conducteur et conçu de telle sorte que le courant d'alimentation parcourt les conducteurs voisins en sens inverse.

5. Mat de bobines selon une des revendications 1 à 4, **caractérisé en ce qu'**au moins un tronçon de conducteur d'une spire de conducteur se compose de plusieurs conducteurs séparés mais couplés électriquement en parallèle et formant un mat de conducteurs, disposés à plat les uns à côté des autres dans le mat en formant des spires de conducteur qui se chevauchent.

6. Mat de bobines selon la revendication 5, **caractérisé en ce que** deux mats de conducteurs ainsi formés sont prévus l'un sur l'autre dans le mat de bobines, décalés latéralement en ce qui concerne les conducteurs.

7. Mat de bobines selon la revendication 6, **caractérisé en ce que** les conducteurs retour des deux mats de conducteurs sont placés très proches l'un de l'autre.

8. Mat de bobines selon une des revendications 5 à 7, **caractérisé en ce que** la section métallique du conducteur retour de chaque mat de conducteurs correspond à la section totale des conducteurs couplés électriquement en parallèle du mat de conducteurs correspondant.

9. Mat de bobines selon une des revendications 6 à 8, **caractérisé en ce que** les deux mats de conducteurs ont un conducteur retour commun.

10. Mat de bobines selon une des revendications précédentes, **caractérisé en ce que** les conducteurs aller et retour de chaque spire de conducteur ont au moins approximativement le même nombre de conducteurs monofilaires.

11. Mat de bobines selon une des revendications 6 à 10, **caractérisé en ce que** les deux mats de conducteurs ont au moins presque la même structure, et **en ce que** le décalage latéral des deux mats de conducteurs correspond au moins approximativement à la moitié de l'écart entre conducteurs dans un des deux mats de conducteurs.

12. Mat de bobines selon une des revendications 6 à 11, **caractérisé en ce que** l'écart entre conducteurs dans les zones où une accentuation ou un affaiblissement du champ magnétique est requis est conçu différemment par rapport aux autres zones, de telle sorte que la différence d'intensité du champ magnétique exigée pour cette zone se règle par rapport aux autres zones.

13. Mat de bobines selon la revendication 12, **caractérisé en ce que** l'écart entre conducteurs dans la zone terminale latérale de chaque mat de conducteurs est réduit de telle sorte que la chute d'intensité du champ magnétique qui se produit normalement dans cette zone pendant le fonctionnement est au moins presque compensée.

14. Mat de bobines selon une des revendications 6 à 13, **caractérisé en ce que** chaque conducteur dans les zones où une accentuation ou un affaiblissement du champ magnétique est requis est conçu différemment quant à sa section métallique et/ou son matériau conducteur, de telle sorte que la différence d'intensité du champ magnétique exigée pour cette zone se règle par rapport aux autres zones lors du fonctionnement.

15. Mat de bobines selon une des revendications 6 à 14, **caractérisé en ce que** le circuit d'alimentation qui connecte les conducteurs à la ligne de raccordement est conçu de telle sorte que les courants dans les conducteurs retour du mat de bobines se compensent, au moins quant au champ magnétique qui en émane.

16. Mat de bobines étendu composé de deux mats de bobines selon une des revendications précédentes, **caractérisé en ce que** les deux mats de bobines sont prévus pour être placés l'un sur l'autre, en se croisant et selon un écart mutuel faible, et **en ce que** chacun des deux mats de bobines est muni d'un raccordement assurant une alimentation indépendante de l'autre mat.

17. Appareil destiné à générer un champ magnétique plan avec un mat de bobines étendu selon la revendication 16 ainsi qu'un générateur destiné à alimenter le mat de bobines, le générateur possédant deux sorties pour alimenter séparément les deux mats de bobines.

18. Appareil selon la revendication 17, **caractérisé en ce que** le générateur est équipé d'un circuit destiné à produire un courant d'alimentation dont les amplitudes ont des caractéristiques prédéfinies en fonction du temps, ce circuit alimentant une des deux sorties, et **en ce qu'**un inverseur de phase relie l'autre sortie au circuit destiné à produire le courant d'alimentation.

19. Appareil selon la revendication 17, **caractérisé en ce que** deux générateurs individuels fournissant un signal de sortie de forme identique sont prévus, chacun alimentant une des deux sorties, et **en ce que** la position de phase entre les signaux de sortie des deux générateurs individuels est modifiable.

20. Appareil selon la revendication 19, **caractérisé en ce que** les générateurs individuels sont conçus comme générateurs de fonctions déclenchables quant à l'instant de démarrage de leur signal de sortie, et **en ce qu'**un circuit d'émission de signaux de déclenchement décalés dans le temps est prévu pour les deux générateurs individuels, l'émission étant de préférence réglable.
